# EUROPÄISCHE PATENTSCHRIFT

(11) **EP 3 334 478 B1**
(45) Veröffentlichungstag und Bekanntmachung des Hinweises auf die Patenterteilung: **28.09.2022**
(21) Anmeldenummer: 16757557.0
(22) Anmeldetag: 10.08.2016
(51) Int. Cl.: A61M 1/28, G01G 23/00, A61M 1/16

(54) **PERITONEALDIALYSEGERÄT**
PERITONEAL DIALYSIS DEVICE
APPAREIL DE DIALYSE PÉRITONÉALE

(30) Priorität: 11.08.2015 DE 102015010418
(43) Veröffentlichungstag der Anmeldung: 20.06.2018
(73) Patentinhaber: Fresenius Medical Care Deutschland GmbH, 61352 Bad Homburg (DE)
(72) Erfinder: ZEYHER, Peter, 64289 Darmstadt (DE)
(74) Vertreter: Herrmann, Uwe
(86) Internationale Anmeldenummer: PCT/EP2016/001381
(87) Internationale Veröffentlichungsnummer: WO 2017/025198

(56) Entgegenhaltungen:
- DE-U1- 9 406 898
- US-A- 4 479 561
- US-A1- 2005 045 388
- US-A1- 2006 289 207
- US-A1- 2011 160 649

## Beschreibung

Die vorliegende Erfindung betrifft ein Peritonealdialysegerät mit wenigstens einem Gerätegehäuse und mit wenigstens einer Wägeschale zur Aufnahme eines oder mehrerer Lösungsbeutel, wobei die Wägeschale mit wenigstens einer Wägezelle zur Gewichtsmessung in Verbindung steht.

Bei aus dem Stand der Technik bekannten Peritonealdialysegeräten befindet sich oberhalb des Gerätegehäuses eine Schale, die zur Aufnahme eines Beutels dient, der mit Dialysat gefüllt ist, das dem Patienten zugeführt wird. Diese Schale - im Folgenden auch als Wägeschale bezeichnet - steht über einen Verbindungsmechanismus, wie z.B. eine Stange mit einer Wägezelle in Verbindung, die sich in oder an dem Gerätegehäuse befindet. Die Wägezelle ermittelt das Gewicht der Wägeschale bzw. des darin befindlichen Beutels.

Bei unsachgemäßer Betätigung, Überladung oder ungleichmäßiger Lastverteilung auf der Wägeschale kann der Fall eintreten, dass die Wägezelle überlastet und dabei ggf. beschädigt wird.

Die US 2011 / 160 649 A1 offenbart einen APD cycler mit einer Wägeschale und mit einem Arretierungssystem, das in einem Zustand die Wägezelle freigibt, so dass diese eine Gewichtsmessung durchführen kann und das in einem weiteren Zustand eine Arretierung derart vornimmt, dass die Wägezelle keine Gewichtsmessung durchzuführen vermag.

Die US 2005 / 045 388 A1 offenbart ein Wägesystem mit einer Wägezelle sowie mit einem integrierten Drehmomentbegrenzer, der gegen in tangentialer Richtung wirkende Momente wirkt.

Die US 2006 / 289 207 A1 offenbart ein Wägesystem mit einer Wägezelle und mit einem integrierten Drehmomentbegrenzer.

Der vorliegenden Erfindung liegt daher die Aufgabe zugrunde, ein Peritonealdialysegerät der eingangs genannten Art dahingehend weiterzubilden, dass eine Überlastung der Wägezelle auf vergleichsweise einfache Art und Weise zuverlässig verhindert wird.

Diese Aufgabe wird durch ein Peritonealdialysegerät der eingangs genannten Art dadurch gelöst, dass eine oder mehrere Drehmomentbegrenzungseinrichtungen vorgesehen sind, die das Auftreten eines Drehmoments oder eines einen Grenzwert übersteigenden Drehmoments in der Wägezelle verhindern, wobei die Drehmomentbegrenzungseinrichtung derart ausgebildet ist, dass diese bei einem auf die Wägeschale wirkenden Drehmoment die Wägeschale derart bewegt, dass diese mit dem Gerätegehäuse oder mit wenigstens einem mit dem Gerätegehäuse in Verbindung stehenden Element in Kontakt tritt, so dass das Drehmoment in das Gerätegehäuse bzw. in das Element abgeleitet wird.

Die Erfindung wird durch die Merkmale des unabhängigen Anspruchs 1 definiert.

Der vorliegenden Erfindung liegt somit der Gedanke zugrunde, einen Schutz der Wägezelle des Peritonealdialysegerätes dadurch zu bewirken, dass bei Auftreten oder bei Überschreiten eines bestimmten Drehmomentes Kräfte von der Wägeschale in das Gerätegehäuse oder in ein damit unmittelbar oder mittelbar in Verbindung stehendes Element abgeleitet werden, so dass keine Beaufschlagung der Wägezelle mit Kräften bzw. Momenten erfolgt oder keine über die zulässige Beaufschlagung der Wägezelle übersteigenden Kräfte bzw. Momente auf diese einwirken.

Bei dem Element kann es sich um ein beliebiges Element des Peritonealdialysegerätes handeln. Vorzugsweise handelt es sich um ein auf der Oberseite des Gerätegehäuses befindliches Element.

Die Bewegung der Wägeschale kann beispielsweise nach oben oder nach unten oder auch in seitlicher Richtung erfolgen. Wesentlich ist, dass die Wägeschale bei bzw. ab einem bestimmten Drehmoment mit dem Gerätegehäuse oder mit dem genannten Element in mechanischen Kontakt tritt und sich somit gewissermaßen an dem Gerätegehäuse bzw. an dem Element und vorzugsweise an der Gehäuseoberseite abstützt. Weitere Kräfte oder Momente werden dann durch das Gerätegehäuse bzw. durch das Element abgeleitet und müssen nicht von der Wägezelle aufgenommen werden, die dementsprechend geschützt wird.

Vorzugsweise ist vorgesehen, dass die Drehmomentbegrenzungseinrichtung derart ausgebildet ist, dass diese erst bei einem auf die Wägeschale wirkenden und einen Grenzwert übersteigenden Drehmoment, die Wägeschale derart bewegt, dass diese mit dem Gerätegehäuse oder mit einem mit dem Gerätegehäuse in Verbindung stehenden Element in Kontakt tritt, so dass das Drehmoment in das Gerätegehäuse bzw. in das Element abgeleitet wird. Der Kontakt zwischen der Wägeschale und dem Gerätegehäuse / Element tritt vorzugsweise somit nicht bei jedem beliebigen auftretenden Moment auf, sondern erst dann, wenn das Moment einen Grenzwert übersteigt.

Erfindungsgemäß ist vorgesehen, dass die Drehmomentbegrenzungseinrichtung wenigstens ein erstes Element und wenigstens ein zweites Element aufweist, wobei das erste Element durch zumindest einen Körper gebildet wird, der wenigstens eine Ausnehmung aufweist, von der wenigstens eine schräge Fläche gegenüber der Horizontalen und gegenüber der Vertikalen geneigt verläuft. Das wenigstens eine zweite Element ist in der Ausnehmung aufgenommen und ist relativ zu dem ersten Element drehbar. Erfolgt eine Drehung der beiden Elemente relativ zueinander, bewegt sich das zweite Element entlang der schrägen Fläche, was zur Folge hat, dass die Wägeschale auf das Gerätegehäuse zu oder von diesem weg bewegt wird.

Die Relativbewegung bzw. -drehung der beiden Elemente zueinander kann beispielsweise auftreten, wenn versucht wird, das Peritonealdialysegerät anhand der Wägeschale zu drehen. Ist das erste Element mittelbar oder unmittelbar an der Wägeschale und das zweite Element mittelbar oder unmittelbar an dem Gerätegehäuse angeordnet, kommt es dann zu einer Relativdrehung der beiden Elemente zueinander, was zu einem Eingreifen der Drehmomentbegrenzungseinrichtung führt. In diesem Fall tritt ab einem bestimmten Drehmoment die Wägeschale in Kontakt z.B. mit der Oberseite des Gerätegehäuses, so dass die auf die Wägeschale einwirkenden Kräfte und Momente in das Gerätegehäuse abgeleitet werden.

Vorzugsweise ist vorgesehen, dass die Ausnehmung wenigstens zwei der geneigten Flächen aufweist, die aufeinander zu laufen.

In der Draufsicht auf das erste Element können die schrägen Flächen des ersten Elementes die Schenkel eines Dreieckes bilden, dessen eine Seite offen ist, damit das zweite Element in die Ausnehmung aufgenommen werden kann. Bei der offenen Seite kann es sich beispielsweise um die oben liegende oder um die unten liegende Seite des Dreiecks handeln.

Anstatt eines Dreiecks sind auch beliebige andere geometrische Formen von der Erfindung mit umfasst.

Das zweite Element, das entlang der wenigstens einen schrägen Fläche entlangbewegt wird, kann beispielsweise als stabförmiges Teil ausgeführt sein.

Das erste oder zweite Element kann drehfest relativ zu der der Wägeschale angeordnet sein und das andere Element kann drehfest relativ zu dem Gerätegehäuse angeordnet sein.

Das erste oder zweite Element kann unmittelbar an der Wägeschale oder unmittelbar an dem Gerätegehäuse angeordnet sein oder auch nur mittelbar mit der Wägeschale oder mit dem Gerätegehäuse in Verbindung stehen.

Denkbar ist es, dass die Wägeschale an einer Befestigungsstange angeordnet ist, deren anderes Ende mit der Wägezelle in Verbindung steht.

Die Drehmomentbegrenzungseinrichtung kann derart ausgebildet sein, dass diese das Drehmoment begrenzt oder die Einwirkung eines Moments auf die Wägezelle ganz verhindert, das bei Rotation der Wägeschale um deren Befestigungsstange entsteht, wobei vorzugsweise vorgesehen ist, dass die Befestigungsstange vertikal verläuft. Von der Erfindung ist jedoch auch eine schräge Anordnung der Befestigungsstange erfasst.

Erfindungsgemäß weist die Drehmomentbegrenzungseinrichtung wenigstens eine Torsionsfeder auf.

Die Torsionsfeder kann plattenförmig ausgeführt sein.

Sie dient vorzugsweise dazu, ein Drehmoment bzw. ein überschüssiges Drehmoment von der Wägezelle abzuhalten, das durch eine Kippbewegung oder Kippbelastung der Wägeschale entsteht. Eine solche liegt vor, wenn die Wägeschale relativ zu ihrem Befestigungspunkt, an dem die Befestigungsstange oder ein sonstiges Befestigungselement ansetzt, ungleichmäßig beladen wird.

Die Torsionsfeder weist einen ersten Abschnitt auf, der mit einem Element der Wägeschale in Verbindung steht. Der erste Abschnitt wird bei einer Kippbewegung der Wägeschale auf Torsion beansprucht.

Der erste Abschnitt kann im Zentrum der Torsionsfeder angeordnet sein und vollumfänglich oder teilweise von einem zweiten Abschnitt umgeben sein, der bei der Kippbewegung nicht oder weniger stark auf Torsion beansprucht wird, wie der erste Abschnitt.

In einer denkbaren Ausgestaltung der Erfindung ist die Torsionsfeder an der Unterseite der Wägeschale angeordnet.

Wie ausgeführt, kann die Drehmomentbegrenzungseinrichtung derart ausgebildet sein, dass diese das auf die Wägezelle wirkende Drehmoment begrenzt oder verhindert, dass auf die Wägezelle ein Drehmoment einwirkt, das bei einer Kippbelastung bzw. Kippbewegung der Wägeschale z.B. um eine horizontale Achse entsteht.

Die Drehmomentbegrenzungseinrichtung kann derart ausgebildet sein, dass diese das auf die Wägezelle wirkende Drehmoment begrenzt oder die Einwirkung eines Drehmoments auf die Wägezelle verhindert, das bei einer Kippbelastung der Wägeschale um eine Kippachse entsteht, die z.B. senkrecht zur Längsachse der Wägeschale verläuft. Weist die Wägeschale eine längliche Form auf, erstreckt sich die Längsachse in Längsrichtung der Wägeschale und die Kippachse im Winkel zu dieser, vorzugsweise senkrecht zu dieser.

Bei der Wägeschale kann es sich um eine beheizte Schale handeln, so dass die Wägeschale als Heizschale ausgebildet ist, in der ein oder mehrere Lösungsbeutel erwärmt werden.

In der Wägeschale kann eine Mehrzahl von Lösungsbeuteln, enthaltend frisches Dialysat angeordnet sein. Denkbar ist es insbesondere, auf einen Heizbeutel zu verzichten, wie er von bekannten Peritonealdialysegeräten bekannt ist und stattdessen eine Mehrzahl von das Dialysat enthaltenden Lösungsbeuteln in der Wägeschale anzuordnen.

Bei bekannten Peritonealdialysegeräten befindet sich in der Wägeschale ein Heizbeutel, der über Schlauchleitungen mit Lösungsbeuteln in Verbindung steht, die das Dialysat enthalten. Von den Lösungsbeuteln gelangt das Dialysat in den Heizbeutel, wird darin erwärmt und gelangt sodann zu dem Patienten.

In einer bevorzugten Ausgestaltung der Erfindung wird auf einen solchen separaten Heizbeutel verzichtet, d.h. das Schlauchset weist keinen Heizbeutel auf. Vielmehr befinden sich alle oder mehrere der Lösungsbeutel, die für eine Behandlung benötigt werden, gleichzeitig in der Wägeschale und werden darin beheizt. Insbesondere bei einer derartigen vergleichsweise hohen Belastung der Heizschale ist darauf zu achten, dass keine Beschädigung der Wägezelle eintritt.

An dieser Stelle wird darauf hingewiesen, dass der Beutel jedes beliebige Aufnahmebehältnis umfasst, das zur Aufnahme einer Lösung geeignet ist. Dieses Behältnis kann starre oder auch flexible Wandungen aufweisen.

Des Weiteren wird darauf hingewiesen, dass der Begriff "Schale" nicht auf eine Schalenform im engeren Sinne begrenzt ist, sondern jede beliebige Aufnahme mit umfasst.

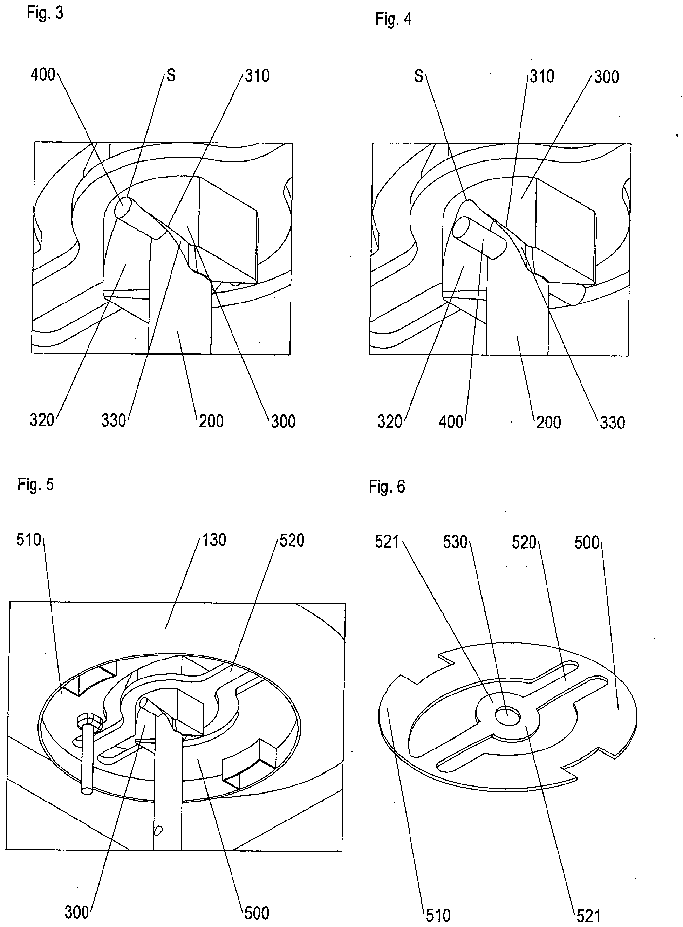

Weitere Einzelheiten und Vorteile der vorliegenden Erfindung werden anhand eines in der Zeichnung dargestellten Ausführungsbeispiels näher erläutert. Es wird an dieser Stelle angemerkt, dass die Figuren 3 und 4 keine erfindungsgemäßen Ausführungsformen darstellen und dass sie zur Klarstellung der vorliegenden Erfindung dienen. Es zeigen:
- Figur 1:: eine perspektivische Ansicht eines Peritonealdialysegerätes gemäß der Erfindung,
- Figur 2:: eine perspektivische Ansicht der Wägeschale von unten,
- Figur 3:: eine vergrößerte perspektivische Ansicht der Wägeschale von unten im Bereich der Drehmomentbegrenzungseinrichtung ohne Drehmomentbeaufschlagung,
- Figur 4:: eine vergrößerte perspektivische Ansicht der Wägeschale von unten im Bereich der Drehmomentbegrenzungseinrichtung mit Drehmomentbeaufschlagung,
- Figur 5:: eine weitere perspektivische Ansicht der Wägeschale von unten mit Torsionsfeder und
- Figur 6:: eine perspektivische Ansicht der Torsionsfeder mit Drehmomentbeaufschlagung.

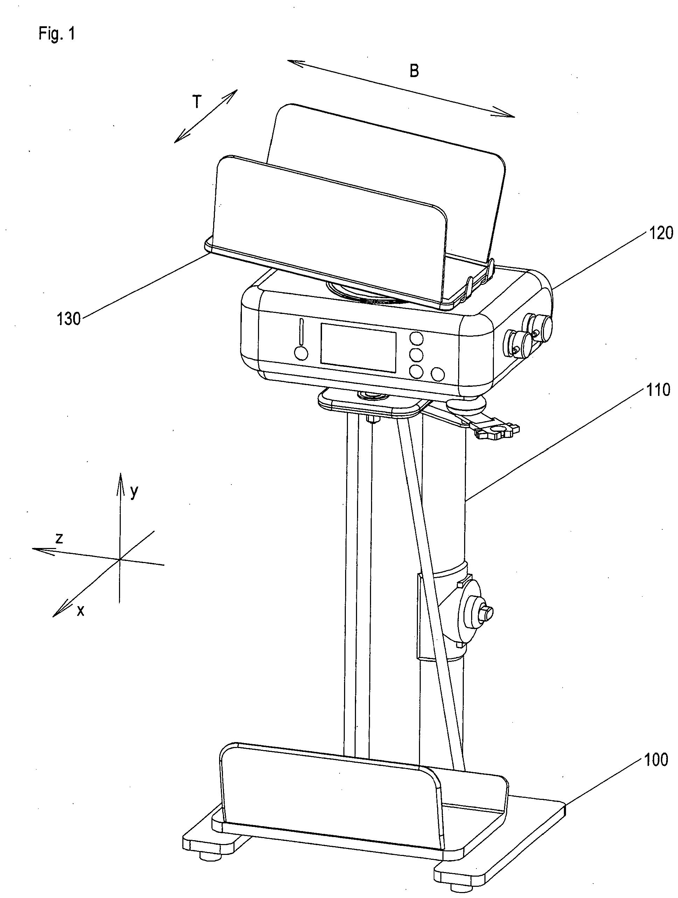

Aus Figur 1 ist ein Peritonealdialysegerät gemäß der Erfindung ersichtlich.

Es weist einen Gerätefuß 100, einen sich von diesem nach oben erstreckenden Gehäuseträger 110 und ein Gerätegehäuse 120 auf, das an dem Gehäuseträger 110 angeordnet ist. In dem Gerätegehäuse 120 befindet sich die für den Betrieb des Gerätes notwendige Steuerung und ggf. Anzeige und/oder Bedienelemente.

Oberhalb des Gerätegehäuses 120 befindet sich die Wägeschale 130, die beheizbar ist und daher im Folgenden als Heizschale 130 bezeichnet wird. Die Heizschale 130 weist eine Breite B und eine Tiefe T auf, wobei die Breite B größer ist als die Tiefe T.

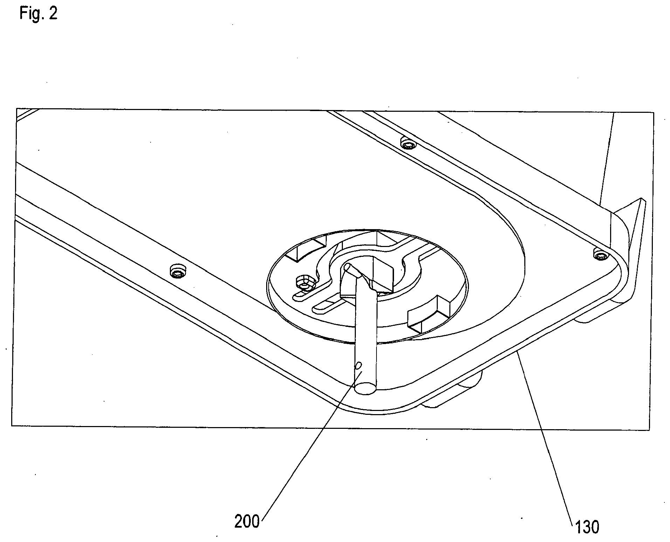

Die Heizschale 130 steht über eine Befestigungsstange 200 mit einer in dem Gerätegehäuse 120 angeordneten Wägezelle in Verbindung, wobei die Befestigungsstange 200 beispielsweise aus Figur 2 ersichtlich ist. Die Befestigungsstange 200 ist vertikal angeordnet.

Gemäß dem in Figur 1 dargestellten Koordinatensystem erstreckt sich die x-Achse in Tiefenrichtung der Heizschale 130, die z-Achse in Breitenrichtung der Heizschale 130 und die y-Achse in Richtung der Befestigungsstange 200, d.h. vertikal nach oben.

Das Peritonealdialysegerät gemäß der vorliegenden Erfindung verfügt über zwei Drehmomentbegrenzungseinrichtungen, die die Aufgabe haben, zu große Drehmomente bzw. überhaupt ein Drehmoment von der Wägezelle abzuhalten und auf diese Weise die Wägezelle zu schützen.

Figur 2 zeigt in einer perspektivischen Ansicht von unten die Heizschale 130. Von der Heizschale 130 nach unten erstreckt sich die Befestigungsstange 200, die die Heizschale 130 trägt und deren Gewicht auf die nicht dargestellte Wägezelle leitet.

Unten an der Heizschale befinden sich zwei Drehmomentbegrenzungseinrichtungen, von denen die erste vergrößert in Figur 3 und in Figur 4 gezeigt ist.

Die erste Drehmomentbegrenzungseinrichtung umfasst ein erstes Element in Form des Körpers 300. Der Körper 300 weist eine im Wesentlichen zylindrische Gestalt auf. Er ist mit der Unterseite der Heizschale fest verbunden, so dass eine Bewegung der Heizschale zu einer entsprechenden Bewegung des Körpers führt.

Auf der Unterseite des Körpers 300 befindet sich ein V-förmiger Einschnitt bzw. eine V-förmige Ausnehmung 310, deren Spitze S nach oben weist und deren untere Seite offen ist.

Die freien Flächen der Ausnehmung 310 sind in Figur 2 mit den Bezugszeichen 320 und 330 gekennzeichnet. Diese Flächen 320, 330 können eben oder gekrümmt ausgeführt sein.

An der Befestigungsstange 200 befindet sich das zweite Element in Form der Stange 400, die sich in einer Richtung senkrecht oder auch schräg zur Längsrichtung der Befestigungsstange 200 erstreckt. Die Stange 400 ist fest an der Befestigungsstange 200 angeordnet. Der Körper 300 ist relativ zu der Befestigungsstange 200 drehbar.

Wie dies aus Figur 3 hervorgeht, befindet sich die Stange 400 am höchsten Punkt, d.h. an der Spitze S der Ausnehmung 300, wenn kein Drehmoment auf die Heizschale 130 einwirkt.

Die Befestigungsstange 200 ist drehfest an dem Gerätegehäuse 120 bzw. an der darin angeordneten Wägezelle angeordnet. Ein auf die Befestigungsstange wirkendes Drehmoment um die Längsachse der Befestigungsstange würde somit auf die Wägezelle übertragen werden.

Die Stange 400 ist drehfest an der Befestigungsstange 200 angeordnet.

Wirkt nun ein Drehmoment auf die Heizschale 130, beispielsweise weil ein Nutzer an dieser dreht, wird dieses Drehmoment nicht auf die Befestigungsstange 200 übertragen. Vielmehr führt das Drehmoment dazu, dass der Körper 300 um die Befestigungsstange 200 gedreht wird. Dies hat zur Folge, dass die Stange 400 aus der Spitze V der Ausnehmung 310 herausbewegt wird und nun - je nach Drehrichtung - mit der Fläche 320 oder 330 der Ausnehmung 310 in Kontakt steht. Dieser Zustand ist in Figur 4 dargestellt.

Die Flächen 320 und 330 können derart gekrümmt sein, dass die Stange über ihre gesamte Länge, in der sie der Ausnehmung 300 aufgenommen ist, stets an der Fläche 320 bzw. 330 anliegt. In diesem Fall ist der Kontakt zwischen der Stange 400 und der Fläche 320 bzw. 330 aufgrund der zylindrischen Form der Stange 400 unabhängig vom Drehwinkel linienförmig.

Von der Erfindung sind auch von einer zylindrischen Form abweichende Formen der Stange umfasst.

Aufgrund der Tatsache, dass die beiden schrägen Flächen 320, 330 nach oben hin aufeinander zulaufen und die Stange 400 ortsfest angeordnet ist, wird aufgrund der Drehbewegung der Heizschale 130 der Körper 300 und somit auch die gesamte Heizschale 130 nach oben bewegt.

Dabei kommt es zu einem Kontakt zwischen der Heizschale 130 und einem Teil der Oberseite des Gerätegehäuses 120. Dieses Teil dient als Anschlag, der eine weitere Drehung der Heizschale 130 verhindert und das Drehmoment in das Gerätegehäuse 120 ableitet.

Anstatt der in Figur 3 und 4 gezeigten Anordnung ist es auch denkbar, den Körper 300 an der Befestigungsstange 200 und die Stange 400 an der Heizschale 130 anzuordnen. In diesem Fall zeigt die offene Seite der Ausnehmung 310 in dem Körper 300 nach oben.

Die Anordnung gemäß Figur 2 bis 4 wirkt bei einem Drehmoment bzw. bei einer Drehung um die y-Achse (vgl. Figur 1), d.h. bei einer Drehung um die Befestigungsstange 200. Da die Heizschale 130 insoweit von der Befestigungsstange 200 entkoppelt ist, folgt die Befestigungsstange 200 nicht der Drehbewegung der Heizschale und erfährt auch kein oder ein allenfalls geringes Drehmoment. Folglich wird auch die Wägezelle nicht mit einem Drehmoment oder nur mit einem geringen Drehmoment beaufschlagt.

Aus Figur 5 ist eine zweite Drehmomentbegrenzungseinrichtung des Peritonealdialysegerätes ersichtlich, die verhindert, dass auf die Wägezelle ein unzulässig hohes Drehmoment wirkt, wenn auf die Heizschale ein Drehmoment um die x-Achse gemäß Figur 1 einwirkt. Dies kann beispielsweise dann der Fall sein, wenn die Heizschale ungleichmäßig mit Heizbeuteln beladen wird.

Wie aus Figur 5 ersichtlich, umfasst die zweite Drehmomentbegrenzungseinrichtung eine plattenförmige Torsionsfeder 500, die sich auf der Unterseite der Heizschale 130 befindet. Die Torsionsfeder 500 als solche ist in Figur 6 gezeigt.

Die Torsionsfeder 500 weist einen äußeren Ring 510 auf, von dem zwei gegenüberliegende Seite durch einen Steg 520 miteinander verbunden sind. In dem Steg 520 befindet sich eine Öffnung 530, die von teilkreisförmigen Teilen 521 des Steges umgeben ist und durch die sich der Körper 300 der ersten Drehmomentbegrenzungseinrichtung erstreckt.

Dies geht beispielsweise aus Figur 5 hervor.

Wirkt nun ein Drehmoment um die durch den Steg 520 gebildete Achse, die der x-Achse in Figur 1 entspricht, wird der Steg 520 in seinem zentralen Abschnitt sowie der die Öffnung 530 umgebende Bereich 521 des Steges 520 durch den sich durch die Öffnung 530 erstreckenden Körper 300 auf Torsion beansprucht und verbogen.

Die Befestigungsstange 200 und die mit dieser in Verbindung stehende Wägezelle erfährt bei dieser Kippbewegung der Heizschale 130 jedoch kein Drehmoment.

Die Biegung dieses Bereiches führt insgesamt zu einer Neigung der Heizschale 130. Sobald die Heizschale 130 einen Teil des Gehäuses berührt, wird eine weitere Verbiegung bzw. ein weiteres Kippen der Heizschale 130 verhindert und das Drehmoment wird in das Gerätegehäuse abgeleitet. Somit wird die Wägezelle nicht nur vor einem Drehmoment bei einer Drehung um die Achse der Befestigungsstange 200 geschützt, sondern auch vor einem Drehmoment, dass bei einer Drehung bzw. bei einem Kippen der Heizschale auftritt.

Exemplarische Beutelgrößen von Beuteln, die auf der Heizschale aufgenommen sind, betragen (Breite x Länge x Höhe, jeweils in cm): 29 x 28 x 8 bei 5 Liter-Beuteln oder 29 x 31 x 10 bei 6 Liter-Beuteln oder 42 x 43 x 4,5 bei 5 Liter-Beuteln oder 42 x 46 x 4,5 bei 6 Liter-Beuteln und hinsichtlich des letzten Beutels 17 x 30 x 7 als 2,5 Liter-Beutel.

Auch andere Beutelgrößen (Volumen und/oder Abmessungen) oder Mehrkammerbeutel sind denkbar und von der Erfindung mit umfasst.

Jeder der vier unterschiedlichen Beutelgrößen bzw. -anordnungen ergeben zusammen mit dem letzten Beutel bei einer Lagerung versetzt zum Lagerungspunkt der Heizschale, d.h. zu dem Punkt, an dem die Befestigungsstange an der Heizschale ansetzt, folgende Drehmomente, die daraus resultieren, dass der Schwerpunkt der beladenen Heizschale nicht mit dem Lagerungspunkt der Heizschale bzw. mit dem Ort der Wägezelle zusammenfällt:
5 Liter-Beutel 29 x 28 x 8 und letzter Beutel bei einem Versatz von 20 mm zur Rechten: 140 N x 0,02 m = 2,8 Nm
6 Liter-Beutel 29 x 31 x 10 und letzter Beutel bei einem Versatz von 15 mm zur Rechten: 160 N x 0,015 m = 2,4 Nm
5 Liter-Beutel 42 x 43 x 4,5 und letzter Beutel bei einem Versatz von 31 mm zur Linken: 140 N x 0,031 m = 4,3 Nm
6 Liter-Beutel 42 x 46 x 4,5 und letzter Beutel bei einem Versatz von 44 mm zur Linken: 160 N x 0,044 m = 7,0 Nm

Die Steifigkeit der Torsionsscheibe bzw. -feder muss so groß sein, dass bei diesen Beladungen noch keine so große Neigung der Heizschale eintritt, dass diese das Gehäuse berührt, da ansonsten keine zuverlässige Messung des Gewichts möglich ist. Der Sicherheitsfaktor zwischen der maximal zu erwartenden Beladung und einer Beladung, die zu einer Berührung der Heizschale auf dem Gehäuse führt, kann im Bereich zwischen 2 und 3, wie z.B. bei 2,5 liegen.

Durch die vorliegende Erfindung ist es möglich, einerseits eine genaue Wägung der Heizschale bzw. der darauf befindlichen Lösungsbeutel zu erzielen und andererseits eine Beschädigung der Wägezelle durch zu große Lasten bzw. Drehmomente zuverlässig zu verhindern.

## Patentansprüche

1. Peritonealdialysegerät mit einem Gerätegehäuse (120) und mit einer Wägeschale (130) zur Aufnahme eines oder mehrerer Lösungsbeutel, wobei die Wägeschale (130) mit einer Wägezelle zur Gewichtsmessung in Verbindung steht,
**dadurch gekennzeichnet,**
**dass** eine oder mehrere Drehmomentbegrenzungseinrichtungen vorgesehen sind, die das Auftreten eines Drehmoments oder eines einen Grenzwert übersteigenden Drehmoments in der Wägezelle verhindern, wobei die Drehmomentbegrenzungseinrichtung derart ausgebildet ist, dass diese bei einem auf die Wägeschale (130) wirkenden Drehmoment die Wägeschale (130) derart bewegt, dass diese mit dem Gerätegehäuse (120) oder mit einem mit dem Gerätegehäuse (120) in Verbindung stehenden Element in Kontakt tritt, so dass das Drehmoment in das Gerätegehäuse (120) bzw. in das Element abgeleitet wird, wobei die Drehmomentbegrenzungseinrichtung ein erstes Element (300) und ein zweites Element (400) aufweist, wobei das erste Element (300) durch einen Körper gebildet wird, der eine Ausnehmung (310) aufweist, von der wenigstens eine Fläche (320, 330) gegenüber der Horizontalen und gegenüber der Vertikalen geneigt verläuft, und wobei das zweite Element (400) in der Ausnehmung (310) aufgenommen ist, wobei das erste Element (300) relativ zu dem zweiten Element (400) verdrehbar ist und wobei die Drehmomentbegrenzungseinrichtung des Weiteren eine Torsionsfeder (500) aufweist, die einen ersten Abschnitt aufweist, der mit der Wägeschale (130) oder mit einem an der Wägeschale (130) angeordneten Körper in Verbindung steht und bei einer Kippbewegung der Wägeschale auf Torsion beansprucht wird.

2. Peritonealdialysegerät nach Anspruch 1, **dadurch gekennzeichnet, dass** die Drehmomentbegrenzungseinrichtung derart ausgebildet ist, dass diese erst bei einem auf die Wägeschale (130) wirkenden und einen Grenzwert übersteigenden Drehmoment, die Wägeschale (130) derart bewegt, dass diese mit dem Gerätegehäuse (120) oder mit einem mit dem Gerätegehäuse (120) in Verbindung stehenden Element in Kontakt tritt, so dass das Drehmoment in das Gerätegehäuse (120) bzw. in das Element abgeleitet wird.

3. Peritonealdialysegerät nach Anspruch 1 oder 2, **dadurch gekennzeichnet, dass** die Ausnehmung (310) zwei der geneigten Flächen (320, 330) aufweist, die aufeinander zu laufen.

4. Peritonealdialysegerät nach Anspruch 3, **dadurch gekennzeichnet, dass** die zwei geneigten Flächen (320, 330) in der Draufsicht auf das erste Element die Schenkel eines Dreieckes bilden, dessen eine Seite offen zur Einführung des zweiten Elementes ausgeführt ist.

5. Peritonealdialysegerät nach einem der Ansprüche 1 bis 4, **dadurch gekennzeichnet, dass** das zweite Element (400) als stabförmiges Teil ausgeführt ist, das vorzugsweise zylindrisch ausgeführt ist.

6. Peritonealdialysegerät nach einem der Ansprüche 1 bis 5, **dadurch gekennzeichnet, dass** das erste oder zweite Element (300, 400) drehfest relativ zu der der Wägeschale (130) angeordnet ist und dass das andere Element (300, 400) drehfest relativ zu dem Gerätegehäuse (120) angeordnet ist.

7. Peritonealdialysegerät nach einem der vorhergehenden Ansprüche, **dadurch gekennzeichnet, dass** die Drehmomentbegrenzungseinrichtung derart ausgebildet ist, dass diese das Auftreten eines Drehmoments an der Wägezelle verhindert oder dieses begrenzt, wenn die Wägeschale (130) um deren Befestigungsachse gedreht wird, wobei vorzugsweise vorgesehen ist, dass die Befestigungsachse vertikal verläuft.

8. Peritonealdialysegerät nach einem der vorhergehenden Ansprüche, **dadurch gekennzeichnet, dass** die Torsionsfeder plattenförmig ausgeführt ist.

9. Peritonealdialysegerät nach einem der vorhergehenden Ansprüche, **dadurch gekennzeichnet, dass** es sich bei dem Körper, der an der Wägeschale (130) angeordnet ist, um den Körper handelt, der das erste Element (300) bildet.

10. Peritonealdialysegerät nach einem der vorhergehenden Ansprüche, **dadurch gekennzeichnet, dass** der erste Abschnitt im Zentrum der Torsionsfeder (500) angeordnet ist und vollumfänglich oder teilweise von einem zweiten Abschnitt der Torsionsfeder (500) umgeben wird.

11. Peritonealdialysegerät nach einem der vorhergehenden Ansprüche, **dadurch gekennzeichnet, dass** die Torsionsfeder (500) an der Unterseite der Wägeschale (130) angeordnet ist.

12. Peritonealdialysegerät nach einem der vorhergehenden Ansprüche, **dadurch gekennzeichnet, dass** die Drehmomentbegrenzungseinrichtung derart ausgebildet ist, dass diese das auf die Wägezelle wirkende Drehmoment begrenzt oder die Einwirkung eines Drehmoments auf die Wägezelle verhindert, wenn die Wägeschale (130) eine Kippbewegung erfährt.

13. Peritonealdialysegerät nach einem der vorhergehenden Ansprüche, **dadurch gekennzeichnet, dass** es sich bei der Wägeschale (130) um eine beheizte Schale handelt und/oder dass in der Wägeschale (130) eine Mehrzahl von Lösungsbeuteln, enthaltend frisches Dialysat angeordnet ist.

## Claims

1. A peritoneal dialysis machine having a machine housing (120) and having a weighing pan (130) for receiving one or more solution bags, wherein the weighing pan (130) is connected to a weighing cell for weight measurement,
**characterized in that**
one or more torque limiting devices are provided which prevent the occurrence of a torque or of a torque exceeding a limit value in the weighing cell, with the torque limiting device being configured such that, with a torque acting on the weighing pan (130), it moves the weighing pan (130) such that it comes into contact with the machine housing (120) or with an element connected to the machine housing (120) such that the torque is led off into the machine housing (120) or into the element, with the torque limiting device having a first element (300) and a second element (400), with the first element (300) being formed by a body which has a recess (310) of which at least one surface (320, 330) extends at an inclination with respect to the horizontal and with respect to the vertical, and with the second element (400) being received in the recess (310), with the first element (300) being rotatable relative to the second element (400), and with the torque limiting device furthermore having a torsion spring (500) that has a first section which is connected to the weighing pan (130) or to a body arranged at the weighing pan (130) and is subjected to torsion on a tilt movement of the weighing pan.

2. A peritoneal dialysis machine in accordance with claim 1, **characterized in that** the torque limiting device is configured such that it only moves the weighing pan (130) on a torque acting on the weighing pan (130) and exceeding a limit value such that it comes into contact with the machine housing (120) or with an element connected to the machine housing (120) such that the torque is led off into the machine housing (120) or into the element.

3. A peritoneal dialysis machine in accordance with claim 1 or claim 2, **characterized in that** the recess (310) has two of the inclined surfaces (320, 330) which run toward one another.

4. A peritoneal dialysis machine in accordance with claim 3, **characterized in that** the two inclined surfaces (320, 330) form, in the plan view of the first element, the sides of a triangle whose one side is open for the introduction of the second element.

5. A peritoneal dialysis machine in accordance with one of the claims 1 to 4, **characterized in that** the second element (400) is designed as a rod-shaped part which is preferably cylindrical.

6. A peritoneal dialysis machine in accordance with one of the claims 1 to 5, **characterized in that** the first element or second element (300, 400) is arranged rotationally fixedly relative to the weighing pan (130); and **in that** the other element (300, 400) is arranged rotationally fixedly relative to the machine housing (120).

7. A peritoneal dialysis machine in accordance with one of the preceding claims, **characterized in that** the torque limiting device is configured such that it prevent or limits the occurrence of a torque at the weighing cell when the weighing pan (130) is rotated about its fastening axle, with provision preferably being made that the fastening axle extends vertically.

8. A peritoneal dialysis machine in accordance with one of the preceding claims, **characterized in that** the torsion spring is of plate shape.

9. A peritoneal dialysis machine in accordance with one of the preceding claims, **characterized in that** the body that is arranged at the weighing pan (130) is the body that forms the first element (300).

10. A peritoneal dialysis machine in accordance with one of the preceding claims, **characterized in that** the first section is arranged at the center of the torsion spring (500) and is surrounded over its full periphery or partially by a second section of the torsion spring (500).

11. A peritoneal dialysis machine in accordance with one of the preceding claims, **characterized in that** the torsion spring (500) is arranged at the lower side of the weighing pan (130).

12. A peritoneal dialysis machine in accordance with one of the preceding claims, **characterized in that** the torque limiting device is configured such that it limits the torque acting on the weighing cell or prevents the action of a torque on the weighing cell when the weighing pan (130) experiences a tilt movement.

13. A peritoneal dialysis machine in accordance with one of the preceding claims, **characterized in that** the weighing pan (130) is a heated pan; and/or **in that** a plurality of solution bags containing fresh dialyzate are arranged in the weighing pan (130).

## Revendications

1. Appareil de dialyse péritonéale comprenant un boîtier d'appareil (120) et un plateau de pesée (130) destiné à recevoir une ou plusieurs poches de solution, le plateau de pesée (130) étant relié à une cellule de pesée pour la mesure pondérale,
**caractérisé en ce que**
un ou plusieurs dispositifs de limitation de couple sont prévus, qui empêchent l'apparition d'un couple ou d'un couple dépassant une valeur limite dans la cellule de pesée, le dispositif de limitation de couple étant conçu de manière à déplacer, en présence d'un couple agissant sur le plateau de pesée (130), le plateau de pesée (130), de telle manière qu'il vient en contact avec le boîtier d'appareil (120) ou avec un élément relié au boîtier d'appareil (120), de telle sorte que le couple est dérivé dans le boîtier d'appareil (120) ou dans l'élément, le dispositif de limitation de couple comportant un premier élément (300) et un second élément (400), le premier élément (300) étant formé par un corps, qui comporte un évidement (310), à partir duquel au moins une surface (320, 330) s'étend de manière inclinée par rapport à l'horizontale et par rapport à la verticale, et le second élément (400) étant reçu dans l'évidement (310), le premier élément (300) pouvant tourner par rapport au second élément (400) et le dispositif de limitation de couple comportant en outre un ressort de torsion (500), qui comporte une première partie reliée au plateau de pesée (130) ou à un corps disposé sur le plateau de pesée (130) et qui est sollicité en torsion lors d'un mouvement basculant du plateau de pesée.

2. Appareil de dialyse péritonéale selon la revendication 1, **caractérisé en ce que** le dispositif de limitation de couple est conçu de manière à déplacer le plateau de pesée (130) uniquement en présence d'un couple agissant sur le plateau de pesée (300) et dépassant une valeur limite, de telle manière que le plateau de pesée vient en contact avec le boîtier d'appareil (120) ou avec un élément relié au boîtier d'appareil (120), de telle sorte que le couple est dérivé dans le boîtier d'appareil (120) ou dans l'élément.

3. Appareil de dialyse péritonéale selon la revendication 1 ou 2, **caractérisé en ce que** l'évidement (310) comporte deux des surfaces (320, 330) inclinées, qui s'étendent l'une vers l'autre.

4. Appareil de dialyse péritonéale selon la revendication 3, **caractérisé en ce que** les deux surfaces (320, 330) inclinées forment, dans la vue de dessus sur le premier élément, les côtés d'un triangle dont un côté est réalisé ouvert pour introduire le second élément.

5. Appareil de dialyse péritonéale selon l'une des revendications 1 à 4, **caractérisé en ce que** le second élément (400) est réalisé sous la forme d'une pièce en forme de tige, qui est de préférence réalisée cylindrique.

6. Appareil de dialyse péritonéale selon l'une des revendications 1 à 5, **caractérisé en ce que** le premier ou le second élément (300, 400) est disposé bloqué en rotation par rapport au plateau de pesée (130) et **en ce que** l'autre élément (300, 400) est disposé bloqué en rotation par rapport au boîtier d'appareil (120).

7. Appareil de dialyse péritonéale selon l'une des revendications précédentes, **caractérisé en ce que** le dispositif de limitation de couple est conçu de manière à empêcher l'apparition d'un couple sur la cellule de pesée ou à limiter celle-ci quand le plateau de pesée (130) est tourné sur son axe de fixation, l'axe de fixation étant de préférence prévu de façon à s'étendre verticalement.

8. Appareil de dialyse péritonéale selon l'une des revendications précédentes, **caractérisé en ce que** le ressort de torsion est réalisé en forme de plaque.

9. Appareil de dialyse péritonéale selon l'une des revendications précédentes, **caractérisé en ce que** le corps qui est disposé sur le plateau de pesée (130) est le corps qui forme le premier élément (300).

10. Appareil de dialyse péritonéale selon l'une des revendications précédentes, **caractérisé en ce que** la première partie est disposée au centre du ressort de torsion (500) et est entourée sur toute la périphérie ou en partie par une seconde partie du ressort de torsion (500).

11. Appareil de dialyse péritonéale selon l'une des revendications précédentes, **caractérisé en ce que** le ressort de torsion (500) est disposé sur la face inférieure du plateau de pesée (130).

12. Appareil de dialyse péritonéale selon l'une des revendications précédentes, **caractérisé en ce que** le dispositif de limitation de couple est réalisé de manière à limiter le couple agissant sur la cellule de pesée ou empêcher l'action d'un couple sur la cellule de pesée quand le plateau de pesée (130) effectue un mouvement basculant.

13. Appareil de dialyse péritonéale selon l'une des revendications précédentes, **caractérisé en ce que** le plateau de pesée (130) est un plateau chauffé et/ou **en ce qu'**une pluralité de poches de solution, contenant du dialysat propre, est disposée dans le plateau de pesée (130)
